(19) Europäisches Patentamt
European Patent Office
Office européen des brevets

(11) Publication number: 0 083 222 B1

(12) **EUROPEAN PATENT SPECIFICATION**

(45) Date of publication of patent specification: **26.02.86**

(51) Int. Cl.[4]: **C 07 D 215/12**, C 07 D 215/18, C 07 D 215/22, C 07 D 221/20, A 61 K 31/47

(21) Application number: **82306916.6**

(22) Date of filing: **23.12.82**

(54) **New quinolylacetic acid compounds and pharmaceutical compositions containing them.**

(30) Priority: **29.12.81 JP 211626/81**

(43) Date of publication of application:
**06.07.83 Bulletin 83/27**

(45) Publication of the grant of the patent:
**26.02.86 Bulletin 86/09**

(84) Designated Contracting States:
**AT BE CH DE FR GB IT LI LU NL SE**

(56) References cited:
**FR-A-2 278 338**
**FR-A-2 395 262**
**GB-A-1 265 648**

**CHEMICAL ABSTRACTS, vol. 64, no. 12, 6th June 1966, column 17538c, Columbus, Ohio, USA H. BOJARSKA-DAHLIG: "6-Quinolyacetic and 6-(1,2,3,4-tetrahydroquinolyl) acetic acid derivatives"**

(73) Proprietor: **NIPPON ZOKI PHARMACEUTICAL CO. LTD.**
**10, 2-chome Hirano-machi Higashi-ku**
**Osaka (JP)**

(72) Inventor: **Hamada, Masaaki**
**10, 2-chome, Hiranomachi**
**Higashi-ku Osaka (JP)**
Inventor: **Okamoto, Kaoru**
**10, 2-chome, Hiranomachi**
**Higashi-ku Osaka (JP)**
Inventor: **Kurosaki, Teikichi**
**10, 2-chome, Hiranomachi**
**Higashi-ku Osaka (JP)**

(74) Representative: **Crampton, Keith John Allen et al**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 083 222 B1

(56) References cited:
CHEMICAL ABSTRACTS, vol. 69, no. 11, 9th September 1968, page 4104, no. 43814k, Columbus, Ohio, USA

CHEMICAL ABSTRACTS, vol. 70, no. 3, 20th January 1969, page 149, no. 10292f, Columbus, Ohio, USA S. KURZEPA et al.: "Influence of some 6-quinolylacetic acid and 6-(1,2,3,4-tetrahydroquinolyl) acetic acid derivatives on the MAO (mono-amine oxidase) activity"

**Description**

This invention is concerned with quinolylacetic acid compounds and pharmaceutical compositions containing them.

GB—A—1 265 648 discloses (1,2,3,4-tetrahydro-6-quinolyl)acetic acid derivatives substituted in at least one of the positions 1—5, 7, 8 and α, in which any substituents present in the α- and/or 1-position are lower aliphatic or cycloaliphatic hydrocarbon residues, any substituents in positions 2, 3 or 4 are lower alkyl groups and any substituents in positions 5, 7 or 8 are selected from lower alkyl groups, halogen atoms and trifluoromethyl, nitrile, nitro, amino, acylamino, hydroxyl, alkoxy, sulphanyl, free mercapto, alkylmercapto, alkylsulphonyl and alkylsulphinyl groups, or an ester or derivative thereof in which two hetero-atoms, of which at least one is a nitrogen atom, are bonded with the C-atom of the modified carboxyl group. The compounds are described as being useful as antiphlogistics, although no pharmacological data are given.

Chem. Abs. (1966), *64* (12), 17538c discloses derivatives of (1,2,3,4-tetrahydro-6-quinolyl)acetic acid of formula

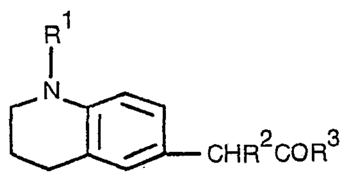

in which $R^1$ is a hydrogen atom or a benzyl or 2-methylpyridyl group; $R^2$ is a hydrogen atom or benzyl group; and $R^3$ is a hydroxy, ethoxy or amide-forming group. These compounds are described as being potential monoamine oxidase inhibitors.

Chem. Abs. (1968), *69* (11), 43814k, discloses derivatives of (1,2,3,4-tetrahydro-6-quinolyl)acetic acid of formula

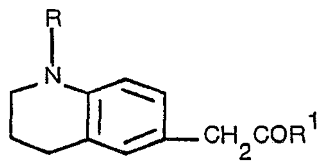

in which R is a hydrogen atom or a benzyl or 2-methylpyridyl group, and $R^1$ is a benzylamino or ethoxy group. These compounds are also described as being useful as monoamine oxidase inhibitors.

Chem. Abs. (1969), *70* (3), 10292f discloses *inter alia* derivatives of (1,2,3,4-tetrahydro-5-quinolyl)acetic acid of formula

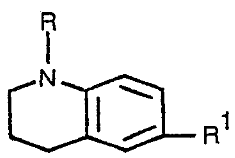

in which R is a hydrogen atom or a benzyl group, and $R^1$ is a group of formula $—CH_2CO_2H$ or an amido derivative thereof which may be substituted at the α-position by a benzyl group or, when R is a benzyl group, $R^1$ may be a hydrogen atom or a methyl group. Compounds of this formula are described as having monoamine oxidase and pig-kidney diamine oxidase inhibitory activity.

FR—A—2 278 338 discloses compounds of formula

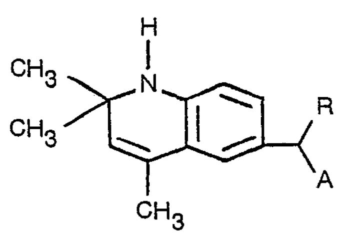

in which R is a hydrogen atom or a methyl group, and A is a group of formula $—COOR_1$ (wherein $R_1$ is a hydrogen atom or an esterifying group), or a group of formula $—CH_2OR_2$ (wherein $R_2$ is a hydrogen atom or an esterifying or etherifying group). These compounds are stated to exhibit anti-inflammatory activity.

FR—A—2 395 262 discloses compounds of formula

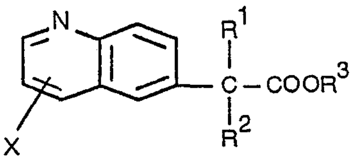

in which $R^1$, $R^2$ and $R^3$ each represents a hydrogen atom or a $C_{1-4}$ alkyl group; and X represents a chlorine atom or a group of formula

OH, $OR^4$, $SR^4$, $SO-R^4$, $SO_2-R^4$, 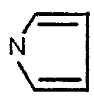 , 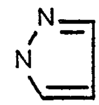 or 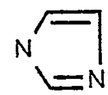

(in which $R^4$ is a $C_{1-4}$ alkyl or phenyl group optionally substituted by a halogen atom or a methyl, alkoxy etc. group). These compounds are also stated to exhibit anti-inflammatory activity.

Although compounds such as salicylates, anthranilic acid derivatives, phenylacetic acid derivatives, indolylacetic acid derivatives and pyrazolones have been used conventionally as drugs having antipyretic, analgesic and antiinflammatory actions, they have given rise to troubles due to side effects such as gastrointestinal, hepatorenal and haematological disorders. Alleviation of these adverse effects has been attempted by improving dosage forms and by chemical modification, or more recently by other methods including the use of pro-drugs.

The present invention results from investigations for developing drugs comparable with or superior to conventional non-steroid drugs in effectiveness, and without the above-mentioned side-effects. As a result, certain new quinolylacetic acid compounds were discovered, and certain of these compounds were found to have antiinflammatory and antipyretic-analgesic actions, as well as low toxicity, thus showing pharmaceutical utility.

The invention therefore provides quinolylacetic acid compounds of the general formula (I)

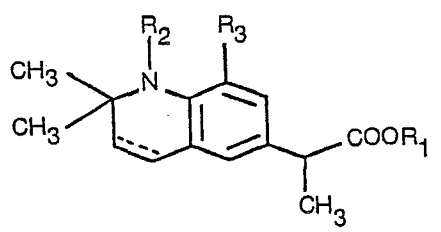

(I)

(in which the broken line indicates an optional double bond between the 3- and 4- positions; $R_1$ is a hydrogen atom or a $C_{1-4}$ alkyl group; $R_2$ is a hydrogen atom or, when the broken line represents a double bond, a methyl or allyl group; and $R_3$ is a halogen atom or, when the broken line represents a double bond, a hydrogen atom; with the proviso that when the broken line represents a double bond, then at least one of $R_2$ and $R_3$ is other than a hydrogen atom) and pharmacologically acceptable salts thereof.

Values of $R_1$ include a hydrogen atom, or straight or branched $C_{1-4}$ alkyl such as methyl, ethyl, propyl, *iso*-propyl, butyl, *iso*-butyl, *sec*-butyl and *t*-butyl group.

Values of $R_3$ include a halogen atom such as fluorine, chlorine, bromine or iodine; and, when the broken line indicates a double bond, a hydrogen atom.

When the broken line is a single bond, the compounds of the present invention have the 1,2,3,4-tetrahydroquinoline structure. When the broken line is a double bond, the compounds of the present invention have the 1,2-dihydroquinoline structure.

Preferred compounds of the present invention are those in which $R_1$ is a hydrogen atom or a methyl or ethyl group; and $R_3$ is a hydrogen, fluorine or chlorine atom. When $R_1$ is a pharmacologically acceptable alkyl group, some advantageous effects are expected, viz. reducing or inhibiting disorders of the gastro duodenum when the compounds are administered by injection or orally, and rapid absorption when they are administered in an ointment. In processes for producing the compounds, the above-mentioned $C_{1-4}$ alkyl groups may be effective as protecting groups for carboxyl acids.

Compounds of the invention will exist as optical isomers and the present invention includes any of the *dl*-, *l*- and *d*-isomers.

The present invention also includes pharmaceutically acceptable salts of the compounds. Salts with alkali metals such as sodium, potassium and lithium, with alkaline-earth metals such as calcium and

magnesium, with other metals such as aluminium, with ammonium, and with organic amines such as trimethylamine, triethylamine, pyrrolidine, piperidine, morpholine, pyridine and picoline, are especially preferred when the compounds are free carboxylic acids.

In accordance with the present invention, the new compounds may be prepared as follows. The substituents are as defined for formula (I) unless otherwise stated.

(1) A compound represented by the general formula (II)

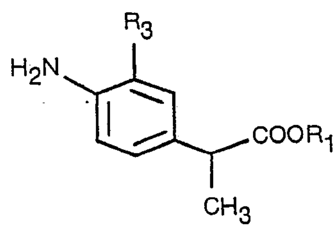

(II)

is reacted with a compound represented by the general formula (III)

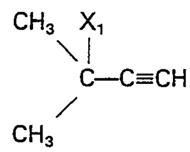

(III)

(where $X_1$ indicates a halogen atom) to give a compound represented by the general formula (IV)

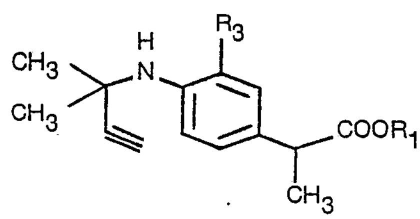

(IV)

and the compound (IV) is converted to a compound represented by the general formula (I—1) by ring closure.

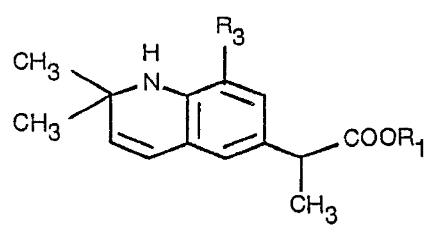

(I—1)

To conveniently carry out the reaction, the compounds represented by the general formula (II) and (III) are allowed to stand at between 0°C and the boiling point of the reaction mixture under reflux in an inert solvent such as tetrahydrofuran, dioxan, diethyl ether, water or mixtures of two or more such solvents, in the presence of a tertiary amine, such as triethylamine, and cuprous chloride and optionally copper powder, to give the compound represented by the general formula (IV).

The compound of the general formula (IV) is an important intermediate compound. It is then heated in an inert solvent such as dioxan, tetrahydrofuran, dimethylformamide or dimethylsulphone, in the presence of a metal halide such as cupric chloride or zinc chloride, to yield by ring closure the intended compound.

(2) A compound represented by the general formula (I—2)

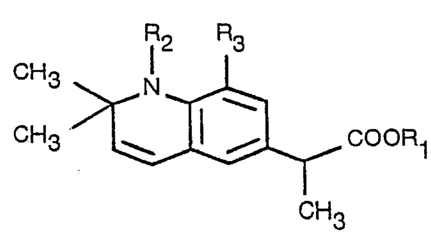

(I—2)

is easily converted to a compound represented by the general formula (I—3) by hydrogenation.

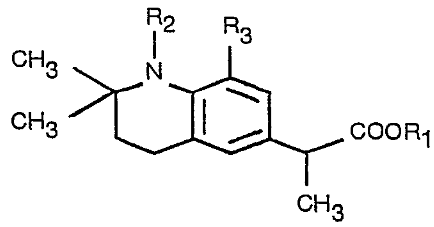

(I—3)

Hydrogenation may conveniently be carried out in an inert solvent, preferably a lower alcohol in the presence of a hydrogenation catalyst such as palladium-on-carbon, palladium black, platinum dioxide or Raney Nickel. It is sufficient to hydrogenate at atmospheric pressure or slightly above, although when the reaction mixture is pressurized and heated, then the reaction takes place more quickly.

(3) A compound represented by the general formula (I—4)

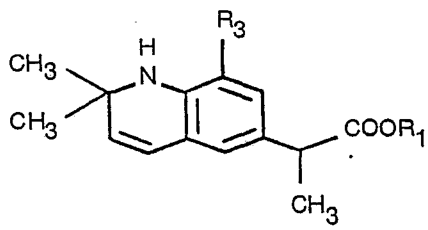

(I—4)

is reacted with a compound represented by the general formula $R_2^*$—$X_2$ (V), where $R_2^*$ is as defined for $R_2$ except that it is not a hydrogen atom and $X_2$ is a halogen atom, a hydroxy group or an active ester group to give a compound represented by the general formula (I—5).

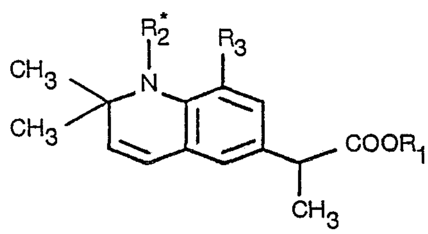

(I—5)

This reaction is conveniently carried out in an inert solvent, preferably a non-protic polar solvent such as dimethylformamide or a ketone, in the presence of a base such as potassium carbonate, with heating at an appropriate temperature. In this case, the yields of the products can be increased by the optional addition of potassium iodide. A basic solvent such as pyridine may also be used.

When $X_2$ is a hydroxy group, the compound (I—4) may be reacted in the presence of a carbodiimide such as dicyclohexylcarbodiimide.

(4) A compound of formula

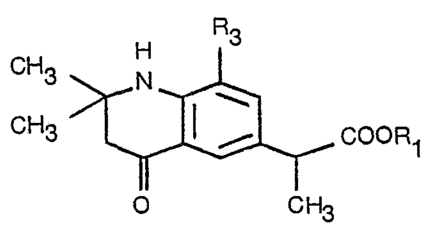

(I—8)

is converted by the Wolf-Kishner reduction, i.e. heating with hydrazine hydrate or an alkaline hydroxide in a solvent such as a glycol or dimethyl sulphoxide, to give a compound represented by the general formula (1—9).

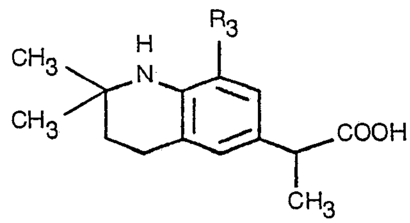

(I—9)

The above-mentioned processes for producing the compounds of the present invention may be used either alone or in combination according to necessity. In each step, the ester derivatives, i.e. compounds in which $R_1$ is an alkyl group, are preferably used. The hydrolysis of the esters to obtain the corresponding free carboxylic acids of the present invention can be effected by well-known processes, carried out in an appropriate solvent such as water, a lower alcohol or mixtures of such solvents, using a base such as sodium hydroxide, potassium hydroxide, sodium carbonate, potassium carbonate, sodium hydrogen carbonate or potassium hydrogen carbonate. In the case of benzyl ester derivatives, the ester residue may be removed by reduction, e.g. in the presence of palladium-on-carbon.

Further, free carboxylic acids of the present invention may be reacted with an alcohol corresponding to the desired $R_1$ in the presence of an acid such as sulphuric acid, hydrogen chloride, hydrochloric acid, *p*-toluenesulfonic acid, camphorsulfonic acid or thionyl chloride to give an ester derivative of the present invention.

The compounds of the present invention also may be converted to their salts, if desired, in the usual ways.

The compounds of the present invention may be purified by usual methods, such as distillation, chromatography and recrystallization. They are identified by, *inter alia,* elemental analysis, melting point, IR, NMR, UV and mass spectroscopy.

The following non-limiting Examples 2—19, which are illustrative, describe the preparation of compounds of the present invention. Example 1 describes the preparation of starting materials.

Example 1

9.3 g of Ethyl 2-(4-amino-3-chlorophenyl)propionate was dissolved in a mixture of 100 ml of dioxan and 30 ml of water. 1.0 g of copper (I) chloride and 10 ml of triethylamine were further added to the solution. 6.3 g of 3-chloro-3-methyl-1-butyn was added dropwise with stirring under a nitrogen atmosphere. The mixture was reacted at 5°C for 2 hours, followed by addition of a mixture of hexane and water and separating the organic layer. The organic layer was washed with water and a saturated aqueous solution of sodium chloride and dried over anhydrous magnesium sulphate; the solvent was then distilled off. The residue was purified by column chromatography on silica gel (eluted with benzene) to give 6.1 g of ethyl 2-[3-chloro-4-(1,1-dimethyl-2-propynylamino)phenyl]propionate (Compound 1).

In the same way, the following compounds were obtained:
Methyl 2-[4-(1,1-dimethyl-2-propynylamino)phenyl]propionate (Compound 2)
Methyl 2-[3-fluoro-4-(1,1-dimethyl-2-propynylamino)phenyl]propionate (Compound 3)

Example 2

6.5 g of Ethyl 2-[3-chloro-4-(1,1-dimethyl-2-propynyl-amino)phenyl]propionate (Compound 1) was dissolved in 70 ml of dioxan, and 10.0 g of zinc chloride was added. The mixture was reacted at 95°C for 15 hours under a nitrogen atmosphere. After the reaction, the mixture was diluted with 100 ml of hexane and subjected to column chromatography on silica gel. The solvent was distilled off to give 6.1 g of ethyl 2-(8-chloro-1,2-dihydro-2,2-dimethylquinolin-6-yl)propionate (Compound 4).

In the same way, the following compound was obtained:
Methyl 2-(8-fluoro-1,2-dihydro-2,2-dimethylquinolin-6-yl)propionate (Compound 5).

Example 3

3 g of Ethyl 2-(8-chloro-1,2-dihydro-2,2-dimethylquinolin-6-yl)propionate (Compound 4) was added to absolute ethanol. Palladium-on-carbon was further added to the solution, and the mixture was reacted with bubbling hydrogen gas at room temperature for 4 hours with stirring. After the reaction, the catalyst was filtered off and ethanol was distilled off to give ethyl 2-(8-chloro-1,2,3,4-tetrahydro-2,2-dimethylquinolin-6-yl)propionate (Compound 6).

In the same way, the following compound was obtained:
Methyl 2-(8-fluoro-1,2,3,4-tetrahydro-2,2-dimethylquinolin-6-yl)propionate (Compound 7).

Example 4

1.9 g of methyl 2-(1,2-dihydro-2,2-dimethylquinolin-6-yl)propionate (prepared according to the method of Example 2) was added to 40 ml of acetone. 2.0 g of potassium carbonate and 2.0 ml of methyl iodide were added to the solution, and the mixture was heated and refluxed for 17 hours. After the mixture was

cooled, the mixture was diluted with ether. Then the precipitate was filtered off, the solvent was distilled off to give 1.9 g of methyl 2-(1,2-dihydro-1,2,2-trimethylquinolin-6-yl)propionate (Compound 8).

In the same way as Example 4, the following compounds of general formula (I—5) were obtained from compounds corresponding to general formula (I—4).

Ethyl 2-(8-chloro-1,2-dihydro-1,2,2-trimethylquinolin-6-yl)propionate (Compound 9)
Methyl 2-(8-fluoro-1,2-dihydro-1,2,2-trimethylquinolin-6-yl)propionate (Compound 10)
Methyl 2-(1-allyl-1,2-dihydro-2,2-dimethylquinolin-6-yl)propionate (Compound 11)

Example 5

3.0 g of ethyl 2-(8-chloro-1,2-dihydro-1,2,2-trimethylquinolin-6-yl)propionate (Compound 9) was added to a mixture of 40 ml of ethanol and 10% aqueous solution of sodium hydroxide. The solution was heated and refluxed for 4 hours. Ethanol was distilled off and the reaction mixture was diluted with water and washed with ether. The aqueous layer was made weakly acidic with hydrochloric acid and then extracted with ethyl acetate. The organic layer was washed with water and a saturated aqueous solution of sodium chloride, and dried over anhydrous magnesium sulfate. The solvent was distilled off, the residue was recrystallized from hexane to give 2.3 g of 2-(8-chloro-1,2-dihydro-1,2,2-trimethylquinolin-6-yl)propionic acid (Compound 12).

In the same way, the following free carboxylic acids were obtained from corresponding ester derivatives.

2-(8-Chloro-1,2-dihydro-2,2-dimethylquinolin-6-yl)propionic acid (Compound 13)
2-(8-Chloro-1,2,3,4-tetrahydro-2,2-dimethylquinolin-6-yl)propionic acid (Compound 14)
2-(1,2-Dihydro-1,2,2-trimethylquinolin-6-yl)propionic acid (Compound 15)
2-(8-Fluoro-1,2-dihydro-2,2-dimethylquinolin-6-yl)propionic acid (Compound 16)
2-(8-Fluoro-1,2,3,4-tetrahydro-2,2-dimethylquinolin-6-yl)propionic acid (Compound 17)
2-(8-Fluoro-1,2-dihydro-1,2,2-trimethylquinolin-6-yl)propionic acid (Compound 18)
2-(1-Allyl-1,2-dihydro-2,2-dimethylquinolin-6-yl)propionic acid (Compound 19)

The characteristic data of compounds of the present invention are listed in Table 1.

TABLE 1 (Oily compound)

| Compound | N M R ($CDCl_3$, δ value) |
|---|---|
| 1 | 1.20(t, 3H), 1.44(d, 3H), 1.64(s, 6H), 2.37(s, 1H), 3.56(q, 1H), 4.08(q, 2H), 4.30(bs, 1H), 6.95 ~ 7.40(m, 3H) |
| 2 | 1.45(d, 3H), 1.57(s, 6H), 2.35(s, 1H), 3.61(q, 1H), 3.63(s, 3H), 3.70(bs, 1H), 6.75 ~ 7.20(m, 4H) |
| 3 | 1.44(d, 3H), 1.60(s, 6H), 2.36(s, 1H), 3.60(q, 1H), 3.63(s, 3H), 3.66(bs, 1H), 6.80 ~ 7.40(m, 3H) |
| 4 | 1.19(t, 3H), 1.31(s, 6H), 1.43(d, 3H), 3.48(q, 1H), 4.09(q, 2H), 4.15(d, 1H), 5.45(dd, 1H), 6.18(d, 1H), 6.70(d, 1H), 6.97(d, 1H) |
| 5 | 1.30(s, 6H), 1.42(d, 3H), 3.58(q, 1H), 3.60(s, 3H), 5.45(d, 1H), 6.18(dd, 1H), 6.55 ~ 6.85(m, 2H), 3.75(bs, 1H) |
| 6 | 1.20(t, 3H), 1.22(s, 6H), 1.41(d, 3H), 1.68(t, 2H), 2.76(t, 2H), 3.51(q, 1H), 4.10(q, 2H), 6.80(d, 1H), 7.00(d, 1H) |
| 7 | 1.21(s, 6H), 1.44(d, 3H), 1.37(t, 2H), 2.75(t, 2H), 3.52(q, 1H), 3.62(s, 3H), 3.70(bs, 1H), 6.58 ~ 6.85(m, 2H) |

TABLE 1 (Continued)

| Compound | N M R ($CDCl_3$, δ value) |
|---|---|
| 8 | 1.30(s, 6H), 1.41(d, 3H), 2.75(s, 3H), 3.58(q, 1H), 3.60(s, 3H), 5.37, 6.18(AB, 2H), 6.38(d, 1H), 6.73(d, 1H), 6.95(dd, 1H) |
| 9 | 1.23(s, 6H), 1.24(t, 3H), 1.46(d, 3H), 2.55(s, 3H), 3.60)q, 1H), 4.14(q, 2H), 5.65, 6.38(AB, 2H), 6.88(d, 1H), 7.15(d, 1H) |
| 10 | 1.28(s, 6H), 1.44(d, 3H), 2.86(d, 3H), 3.56(q, 1H), 3.64(s, 3H), 5.54(d, 1H), 6.26(dd, 1H), 6.60 ~ 6.90(m, 2H) |
| 11 | 1.32(s, 6H), 1.42(d, 3H), 3.55(q, 1H), 3.60(s, 3H), 3.76 ~ 3.95(m, 2H), 4.95 ~ 5.40(m, 2H), 5.30, 6.14(AB, 2H), 5.55 ~ 6.00(m, 1H), 6.30 ~ 6.86(m, 3H) |

TABLE 1 (Continued)

| Compound | m.p. (°C) | NMR ($CDCl_3$, δ value) | IR (KBr, $cm^{-1}$) |
|---|---|---|---|
| 12 | 86 ~ 87 | 1.22(s, 6H), 1.48(d, 3H), 2.53(s, 3H), 3.62(q, 1H), 5.65, 6.38(AB, 2H), 6.87(d, 1H), 7.15(d, 1H), 10.50 (bs, 1H) | 3600 ~ 2300, 2960 1705, 1545, 1465 1450, 1405, 1215 890, 725 |
| 13 | 130 ~ 132 | 1.35(s, 6H), 1.37(d, 3H), 3.52(q, 1H), 5.51(d, 1H), 6.22(d, 1H), 6.75(d, 1H), 6.98(d, 1H), 4.5 ~ 6.5 (bs, 2H), (Acetone–$d_6$) | 3600 ~ 2300, 3400 1710, 1490, 1210 665 |
| 14 | 155 ~ 158 | 1.22(s, 6H), 1.44(d, 3H), 1.67(t, 2H), 2.76(t, 2H), 3.54(q, 1H), 6.80 ~ 7.05 (m, 2H), 7.40(bs, 2H) | 3600 ~ 2300, 3420 1710, 1610, 1500 |
| 15 | 128 ~ 131 | 1.30(s, 6H), 1.42(d, 3H), 2.75(s, 3H), 3.54(q, 1H), 5.37, 6.17(AB, 2H), 6.38(d, 1H), 6.75(d, 1H), 6.96(dd, 1H) | 3500 ~ 2300, 1700 1650, 1602, 1495 1320, 1120 |
| 16 | 127 ~ 130 | 1.30(s, 6H), 1.42(d, 3H), 3.52(q, 1H), 5.44(d, 1H), 6.18(dd, 1H), 6.55 ~ 6.85(m, 2H), 7.30(bs, 2H) | 3600 ~ 2300, 3425 1705, 1635, 1580 1495, 1230, 725 670 |
| 17 | 154 ~ 156 | 1.24(s, 6H), 1.36(d, 3H), 1.68(t, 2H), 2.76(t, 2H), 3.56(q, 1H), 4.95(bs, 2H), 6.60 ~ 6.90 (m, 2H) (Acetone–$d_6$) | 3600 ~ 2300, 3440 1705, 1625, 1585 1510, 1230, 1160 865, 675 |
| 18 | 95 ~ 97 | 1.28(s, 6H), 1.44(d, 3H), 2.87(d, 3H), 3.56(q, 1H), 5.52(d, 1H), 6.28(dd, 1H), 6.60 ~ 6.95(m, 2H), 10.70 (bs, 1H) | 3600 ~ 2300, 1705 1560, 1230 |
| 19 | 98 ~ 100 | 1.32(s, 6H), 1.41(d, 3H), 3.52(q, 1H), 3.75~3.95(m, 2H), 4.90~5.35(m, 2H), 5.45~6.00 (m, 1H), 5.70, 6.12(AB, 2H), 6.27~6.95(m, 3H), 10.30(bs, 1H) | 3600 ~ 2300, 2970 1700, 1605, 1495 1335 |

Compounds of the present invention, which have new structures different from those of conventional non-steroid anti-inflammatory, analgesic and antipyretic compounds, have been found to be of low toxicity and to be very powerful in the desired activities.

The following serves to illustrate animal studies.

(1) Acute toxicity

Groups of 10 male dd mice each weighing some 18 g were orally administered the examined drugs and the number of deaths up to 72 hours later was recorded.

Results are shown in Table 2.

TABLE 2

| Drugs | dosage (mg/kg) | Mortality (%) |
|---|---|---|
| Aminopyrine | 600 | 20 |
| | 800 | 100 |
| Phenylbutazone | 400 | 30 |
| | 600 | 100 |
| Compound 13 | 400 | 0 |
| | 800 | 20 |
| Compound 14 | 400 | 0 |
| | 800 | 10 |

(2) Inhibition of plantar oedema induced by carrageenin

60 minutes after the drugs had been orally administered to groups of 7 Wister rats, 0.1 ml of 1% carrageenin solution was injected subcutaneously into the sole. The volume of the hind foot was determined thereafter at intervals. The rate of increase in volume compared with the volume before carrageenin treatment was regarded as the oedema rate.

An example of the results is shown in the single figure of the drawings.

Furthermore, sections of the stomachs and intestinal tracts of the rats, after the above-mentioned observations had been made, revealed mucosal disorder or ulcer formation in the phenylbutazone-administered group, while no abnormality was found in any animals in the group that received the compounds of the present invention.

(3) Analgesic effects by a modified Haffner method

Groups of 10 dd mice were orally administered the examined drugs and 30 minutes later 2 mg/kg of morphine hydrochloride, the threshold dose, was injected subcutaneously. At 15, 30, 45 and 60 minutes thereafter, the root of mouse's tail was squeezed with a Kocher's forceps. The number of animals that did not exhibit pseudopain reactions was recorded and the determination with the largest number of responding animals among the four determinations was used for evaluation.

Results are shown in Table 3.

TABLE 3

| Drugs | Analgesic effects (%) 100 mg/kg | 200 mg/kg |
|---|---|---|
| Aminopyrine | 30 | 35 |
| Compound 13 | 35 | 50 |
| Compound 14 | 30 | 55 |

(4) Analgesic effects by acetic acid writhing test

30 minutes after the examined drug was orally given 0.1 ml/10 g of 0.6% acetic acid solution was intraperitoneally administered to a group of 10 male dd mice each weighing some 18 g. The amount of stretching was calculated during an observation period of 15 to 20 minutes after acetic acid treatment. The average inhibition rate of stretching among 8 animals was regarded as the inhibition rate of the drug, excluding 2 animals showing maximum and minimum numbers of stretching.

Results are shown in Table 4.

TABLE 4

| Drugs | Analgesic effects (%) 50 mg/kg | 100 mg/kg | 150 mg/kg |
|---|---|---|---|
| Aminopyrine | 14 | 47 | 60 |
| Compound 13 | 37 | 52 | 68 |
| Compound 14 | 36 | 61 | 73 |

As the above experiments clearly indicate, compounds of the present invention have excellent antiinflammatory and antipyretic action. Therefore, these compounds can be used therapeutically as antiinflammatory, analgesic and antipyretic agents against various inflammations, such as those in rheumatic diseases, arthritis, and other inflammations having a variety of redness, fever, swelling and pain, against painful diseases and manifestations such as acute and chronic pain, neuralgia and pains accompanied by inflammation, trauma and lumbago, and against a variety of symptoms having fever.

Although the test data presented in this application relate primarily to rats and mice, the compounds of the present invention produce similar results in all mammals such as dogs, cats, monkeys, apes and man.

The compounds of the present invention can be made into pharmaceutical compositions by combination with appropriate medical carriers or diluents, and dosage forms in solid, semisolid, liquid or gaseous form can be prepared in the usual way for oral or non-oral administrations.

In pharmaceutical dosage forms, the compounds of the present invention may be used in the form of their pharmaceutically acceptable salts, and they also can be used alone or in appropriate association, as well as in combination with other pharmaceutically active components.

When the compounds are given orally, they may be used alone or combined with appropriate fillers to make tablets or capsules, e.g. with conventional bases such as lactose, mannitol, corn starch and potato starch, with binders such as crystalline cellulose, cellulose derivatives, acacia, corn starch and gelatin, with disintegrators such as corn starch, potato starch and sodium carboxymethylcellulose, and with lubricants such as talc and magnesium stearate. They also can be combined with ointment bases such as vaseline, paraffin, plastibase, simple ointment, hydrophilic ointment, hydrophilic petrolatum and hydrophilic plastibase to make ointments.

Furthermore, the compounds of the present invention may be mixed thoroughly with a variety of bases such as emulsifying bases or water-soluble bases to give suppositories.

The compounds of the present invention can also be injected as solutions or suspensions in aqueous or non-aqueous solvents such as vegetable oils, synthetic aliphatic acid glycerides, esters of higher aliphatic acids and propylene glycol.

When utilized as inhalation or aerosol preparations, the compounds of the present invention in the form of a liquid or minute powder can be put in an aerosol container, with gas or liquid spraying agents, and with conventional adjuvants such as humidifying or dispersing agents added, if necessary. The compounds of the present invention may also be applied as pharmaceuticals for non-pressurized preparations such as in a nebulizer or an atomizer.

Poultices can be prepared by mixing the compounds with mentha oil, concentrated glycerin, kaolin or other suitable additives.

The desirable dose of the compounds of the present invention varies with the subject, method and period of administration, but generally it is recommended to administer orally 10 to 3,000 mg of these compounds daily to an adult to obtain the desired effects. One to several units of the unit preparation containing the compounds of the present invention in appropriate amount may be administered. As for non-oral administration (e.g. for injectional forms), doses in the order of one tenth to one third of the above oral dose are desirable as daily doses.

Some prescriptions of the pharmaceutical compositions are shown below as examples which contain the compounds of the present invention as active ingredients.

Prescription Example 1. (tablet)

| Components | Content of a tablet (mg) |
|---|---|
| an invented compound | 50 |
| lactose | 145 |
| corn starch | 55 |
| magnesium stearate | 10 |
| | total 260 mg |

Prescription Example 2. (capsule)

| Components | Content of a capsule (mg) |
|---|---|
| an invented compound | 100 |
| lactose | 200 |
| | total 300 mg |

Prescription Example 3. (injection)

| Components | Content of an ampoule (mg) |
|---|---|
| An invented compound | 10 |
| sodium chloride | proper amount |
| distilled water for injection | proper amount |
| | total 1 ml |

Prescription Example 4. (ointment)

| Components | Weight (g) |
|---|---|
| an invented compound | 1 |
| emulsified wax | 30 |
| white petrolatum | 50 |
| liquid paraffin | 20 |
| | total 101 g |

Prescription Example 5. (suppository)

| Components | Content of a suppository (mg) |
|---|---|
| an invented compound | 20 |
| cacao butter | 1980 |
| | total 2000 mg |

**Claims for the Contracting States: BE CH DE FR GB IT LI LU NL SE**

1. Quinolylacetic acid compounds of the general formula (I)

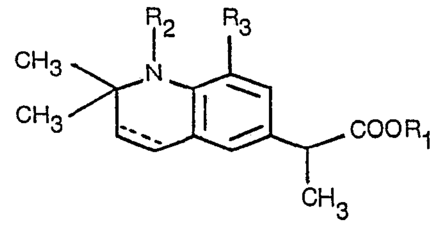

(I)

(in which the broken line indicates an optional double bond between the 3- and 4- positions; $R_1$ is a hydrogen atom or a $C_{1-4}$ alkyl group; $R_2$ is a hydrogen atom or, when the broken line represents a double bond, a methyl or allyl group; and $R_3$ is a halogen atom or, when the broken line represents a double bond, a hydrogen atom; with the proviso that when the broken line represents a double bond, then at least one of $R_2$ and $R_3$ is other than a hydrogen atom) and pharmacologically acceptable salts thereof.

2. 2-(8-Chloro-1,2-dihydro-2,2-dimethylquinolin-6-yl)propionic acid and its pharmaceutically acceptable salts.

3. 2-(8-Chloro-1,2,3,4-tetrahydro-2,2-dimethylquinolin-6-yl)propionic acid and its pharmaceutically acceptable salts.

4. 2-(8-Chloro-1,2-dihydro-1,2,2-trimethylquinolin-6-yl)propionic acid and its pharmaceutically acceptable salts.

5. 2-(8-Fluoro-1,2-dihydro-2,2-dimethylquinolin-6-yl)propionic acid and its pharmaceutically acceptable salts.

6. 2-(8-Fluoro-1,2,3,4-tetrahydro-2,2-dimethylquinolin-6-yl)propionic acid and its pharmaceutically acceptable salts.

7. 2-(8-Fluoro-1,2-dihydro-1,2,2-trimethylquinolin-6-yl)propionic acid, and its pharmaceutically acceptable salts.

8. 2-(1-Allyl-1,2-dihydro-2,2-dimethylquinolin-6-yl)propionic acid, and its pharmaceutically acceptable salts.

9. 2-(1,2-Dihydro-1,2,2-trimethylquinolin-6-yl)propionic acid and its pharmaceutically acceptable salts.

10. A compound as claimed in any one of claims 1 to 9 for use in the treatment of a disease or condition requiring an antiinflammatory, antipyretic or analgesic agent.

11. A composition comprising a carrier and a compound as claimed in any one of claims 1 to 11.

**Claims for the Contracting State: AT**

1. A process for the preparation of quinolylacetic acid compounds of the general formula (I)

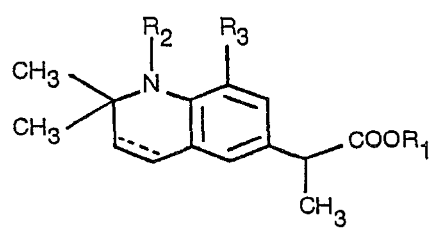

(I)

(in which the broken line indicates an optional double bond between the 3- and 4- positions; $R_1$ is a hydrogen atom or a $C_{1-4}$ alkyl group; $R_2$ is a hydrogen atom or, when the broken line represents a double bond, a methyl or allyl group; and $R_3$ is a halogen atom or, when the broken line represents a double bond, a hydrogen atom; with the proviso that when the broken line represents a double bond, then at least one of $R_2$ and $R_3$ is other than a hydrogen atom) and pharmacologically acceptable salts thereof, which comprises

(a) reacting a compound of formula

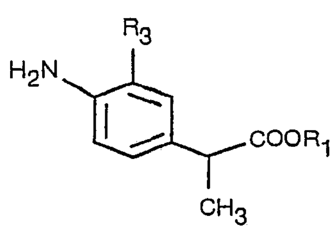

(II)

(wherein $R_1$ and $R_3$ are as defined above) with a compound of formula

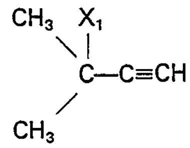

(III)

(wherein $X_1$ represents a halogen atom) to give a compound of formula

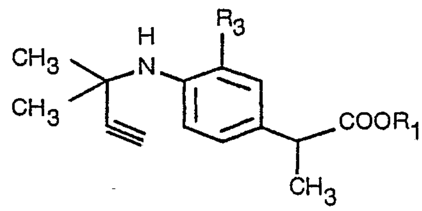

(IV)

(wherein $R_1$ and $R_3$ are as defined above) and effecting ring closure to give a compound of general formula (I);

(b) hydrogenating a compound of general formula

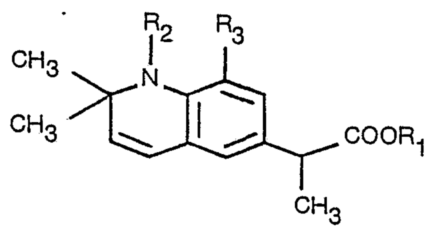

(I–2)

(wherein $R_1$, $R_2$ and $R_3$ are as defined above);

(c) reacting a compound of formula

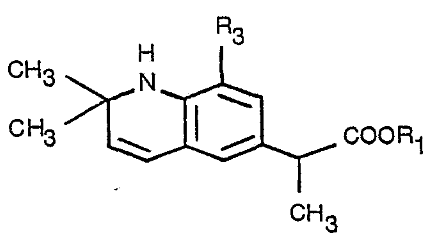

(I–4)

(wherein $R_1$ and $R_3$ are as defined above) with a compound of formula

$$R_2^*\text{—}X_2 \qquad (V)$$

(wherein $R_2^*$ is as defined above for $R_2$ except that it is not a hydrogen atom, and $X_2$ is a halogen atom, a hydroxy group or an active ester group); and

(d) reducing a compound of formula

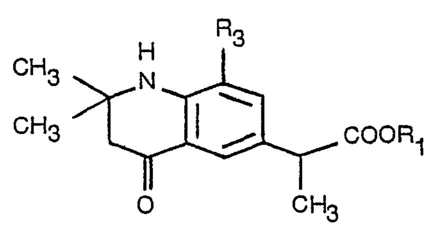

(I–8)

(wherein $R_1$ and $R_3$ are as defined above).

2. A process according to claim 1 for the preparation of 2-(8-chloro-1,2-dihydro-2,2-dimethylquinolin-6-yl)propionic acid and its pharmaceutically acceptable salts.

3. A process according to claim 1 for the preparation of 2-(8-chloro-1,2,3,4-tetrahydro-2,2-dimethylquinolin-6-yl)propionic acid and its pharmaceutically acceptable salts.

4. A process according to claim 1 for the preparation of 2-(8-chloro-1,2-dihydro-1,2,2-trimethylquinolin-6-yl)propionic acid and its pharmaceutically acceptable salts.

5. A process according to claim 1 for the preparation of 2-(8-fluoro-1,2-dihydro-2,2-dimethylquinolin-6-yl)propionic acid and its pharmaceutically acceptable salts.

6. A process according to claim 1 for the preparation of 2-(8-fluoro-1,2,3,4-tetrahydro-2,2-dimethylquinolin-6-yl)propionic acid and its pharmaceutically acceptable salts.

7. A process according to claim 1 for the preparation of 2-(8-fluoro-1,2-dihydro-1,2,2-trimethylquinolin-6-yl)propionic acid, and its pharmaceutically acceptable salts.

8. A process according to claim 1 for the preparation of 2-(1-allyl-1,2-dihydro-2,2-dimethylquinolin-6-yl)propionic acid, and its pharmaceutically acceptable salts.

9. A process according to claim 1 for the preparation of 2-(1,2-dihydro-1,2,2-trimethylquinolin-6-yl)-propionic acid and its pharmaceutically acceptable salts.

10. A process for the preparation of a pharmaceutical composition which comprises admixing a compound of formula (I) as defined in claim 1 or a pharmaceutically acceptable salt thereof with a pharmaceutical carrier or excipient.

**Patentansprüche für die Vertragsstaaten: BE CH DE FR GB IT LI LU NL SE**

1. Chinolylessigsäureverbindungen der allgemeinen Formel (I)

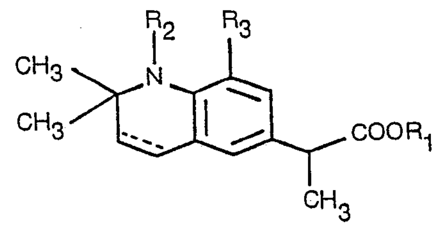

(I)

(worin die gestrichelte Linie gegebenenfalls eine Doppelbindung zwischen der 3- und der 4-Stellung bedeutet, $R_1$ ein Wasserstoffatom oder eine $C_{1-4}$-Alkylgruppe ist, $R_2$ ein Wasserstoffatom oder, wenn die gestrichelte Linie eine Doppelbindung bedeutet, eine Methyl- oder Allylgruppe ist und $R_3$ ein Halogenatom oder, wenn die gestrichelte Linie eine Doppelbindung ist, ein Wasserstoffatom ist, wobei, wenn die gestrichelte Linie eine Doppelbindung bedeutet, wenigstens eine der Gruppen $R_2$ und $R_3$ etwas anderes als ein Wasserstoffatom ist) und pharmakologisch verträgliche Salze hiervon.

2. 2-(8-Chlor-1,2-dihydro-2,2-dimethylchinolin-6-yl)propionsäure und deren pharmazeutisch verträgliche Salze.

3. 2-(8-Chlor-1,2,3,4-tetrahydro-2,2-dimethylchinolin-6-yl)propionsäure und deren pharmazeutisch verträgliche Salze.

4. 2-(8-Chlor-1,2-dihydro-1,2,2-trimethylchinolin-6-yl)propionsäure und deren pharmazeutisch verträgliche Salze.

5. 2-(8-Fluor-1,2-dihydro-2,2-dimethylchinolin-6-yl)propionsäure und deren pharmazeutisch verträgliche Salze.

6. 2-(8-Fluor-1,2,3,4-tetrahydro-2,2-dimethylchinolin-6-yl)-propionsäure und deren pharmazeutisch verträgliche Salze.

7. 2-(8-Fluor-1,2-dihydro-1,2,2-trimethylchinolin-6-yl)propionsäure und deren pharmazeutisch verträgliche Salze.

8. 2-(1-Allyl-1,2-dihydro-2,2-dimethylchinolin-6-yl)propionsäure und deren pharmazeutisch verträgliche Salze.

9. 2-(1,2-Dihydro-1,2,2-trimethylchinolin-6-yl)propionsäure und deren pharmazeutisch verträgliche Salze.

10. Eine Verbindung nach einem der Ansprüche 1 bis 9 für die Verwendung bei der Behandlung einer Erkrankung oder Bedigung, die ein entzündungshemmendes, antipyretisches oder analgetisches Mittel erfordert.

11. Zusammensetzung mit einem Trägermaterial und einer Verbindung nach einem der Ansprüche 1 bis 11.

**Patentansprüche für den Vertragsstaat: AT**

1. Verfahren zur Herstellung von Chinolylessigsäureverbindungen der allgemeinen Formel (I)

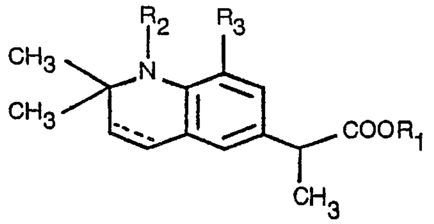

(I)

(worin die gestrichelte Linie gegebenenfalls eine Doppelbindung zwischen der 3- und der 4-Stellung bedeutet, $R_1$ ein Wasserstoffatom oder eine $C_{1-4}$-Alkylgruppe ist, $R_2$ ein Wasserstoffatom oder, wenn die gestrichelte Linie eine Doppelbindung bedeutet, eine Methyl- oder Allylgruppe ist und $R_3$ ein Halogenatom oder, wenn die gestrichelte Linie eine Doppelbindung bedeutet, ein Wasserstoffatom ist, wobei, wenn die gestrichelte Linie eine Doppelbindung ist, wenigstens eine der Gruppen $R_2$ und $R_3$ etwas anderes als ein Wasserstoffatom ist) und pharmakologisch verträglicher Salz hiervon, bei dem man

a) eine Verbindung der Formel

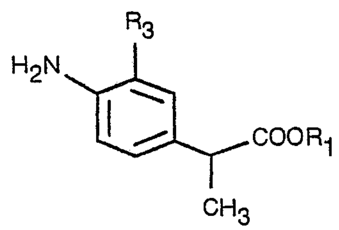

(II)

(worin $R_1$ und $R_3$ wie oben definiert sind) mit einer Verbindung der Formel

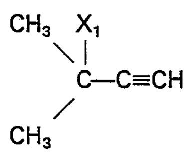

(III)

(worin $X_1$ ein Halogenatom bedeutet) unter Bildung einer Verbindung der Formel

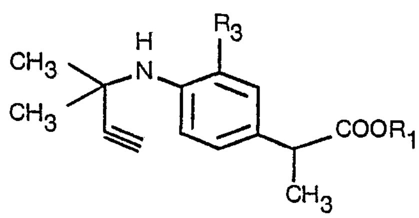

(IV)

(worin $R_1$ und $R_3$ wie oben definiert sind) umsetzt und Ringschluß unter Bildung einer Verbindung der allgemeinen Formel (I) bewirkt, oder

b) eine Verbindung der allgemeinen Formel

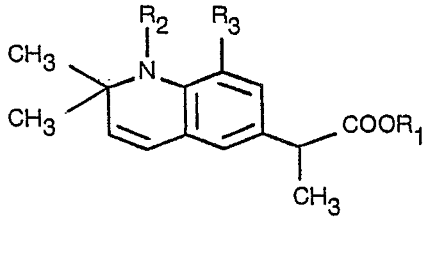

(I–2)

(worin $R_1$, $R_2$ und $R_3$ wie oben definiert sind) hydriert oder

c) eine Verbindung der Formel

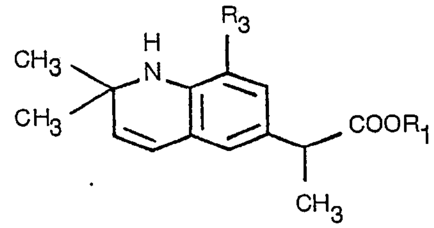

(I–4)

(worin $R_1$ und $R_3$ wie oben definiert sind) mit einer Verbindung der Formel

$$R_2^*—X_2 \quad (V)$$

(worin $R_2^*$ wie oben $R_2$ definiert ist, jedoch mit der Ausnahme, daß es nicht ein Wasserstoffatom ist, und $X_2$ ein Halogenatom, eine Hydroxylgruppe oder eine aktive Estergruppe ist) umsetzt oder

d) eine Verbindung der Formel

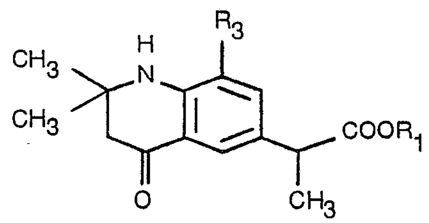

(I–8)

(worin $R_1$ und $R_3$ wie oben definiert sind) reduziert.

2. Verfahren nach Anspruch 1 zur Herstellung von 2-(8-Chlor-1,2-dihydro-2,2-dimethylchinolin-6-yl)-propionsäure und von deren pharmazeutisch verträglichen Salzen.

3. Verfahren nach Anspruch 1 zur Herstellung von 2-(8-Chlor-1,2,3,4-tetrahydro-2,2-dimethylchinolin-6-yl)propionsäure und von deren pharmazeutisch verträglichen Salzen.

4. Verfahren nach Anspruch 1 zur Herstellung von 2-(8-Chlor-1,2-dihydro-1,2,2-dimethylchinolin-6-yl)-propionsäure und von deren pharmazeutisch verträglichen Salzen.

5. Verfahren nach Anspruch 1 zur Herstellung von 2-(8-Fluor-1,2-dihydro-2,2-dimethylchinolin-6-yl)-propionsäure und von deren pharmazeutisch verträglichen Salzen.

6. Verfahren nach Anspruch 1 zur Herstellung von 2-(8-Fluor-1,2,3,4-tetrahydro-2,2-dimethylchinolin-6-yl)propionsäure und von deren pharmazeutisch verträglichen Salzen.

7. Verfahren nach Anspruch 1 zur Herstellung von 2-(8-Fluor-1,2-dihydro-1,2,2-trimethylchinolin-6-yl)-propionsäure und von deren pharemazeutisch verträglichen Salzen.

8. Verfahren nach Anspruch 1 zur Herstellung von 2-(1-Allyl-1,2-dihydro-2,2-dimethylchinolin-6-yl)-propionsäure und von deren pharmazeutisch verträglichen Salzen.

9. Verfahren nach Anspruch 1 zur Herstellung von 2-(1,2-Dihydro-1,2,2-trimethylchinolin-6-yl)propionsäure und von deren pharmazeutisch verträglichen Salzen.

10. Verfahren zur Herstellung einer pharmazeutischen Zusammensetzung durch Vermischen einer Verbindung der Formel (I), wie sie in Anspruch 1 definiert ist, oder eines pharmazeutisch verträglichen Salzes derselben mit einem pharmazeutischen Träger- oder Verdünnungsmaterial.

**Revendications pour les Etats contractants: BE CH DE FR GB IT LI LU NL SE**

1. Composés de l'acide quinolylacétique de formule générale (I)

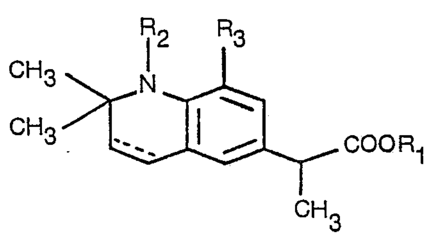

(I)

(dans laquelle le pointillé indique une double liaison éventuelle entre les positions 3 et 4; $R_1$ est un atome d'hydrogène ou un groupe alkyle $C_{1-4}$; $R_2$ est un atome d'hydrogène ou, lorsque le pointillé représente une double liaison, un groupe méthyle ou allyle; et $R_3$ est un atome d'halogène ou, lorsque le pointillé représente une double liaison, un atome d'hydrogène; sous réserve que lorsque le pointillé représente une double liaison, au moins un de $R_2$ et $R_3$ est autre qu'un atome d'hydrogène), et leurs sels acceptables en pharmacologie.

2. Acide 2-(8-chloro-1,2-dihydro-2,2-diméthylquinoléine-6-yl)propionique et ses sels acceptables en pharmacie.

3. Acide 2-(8-chloro-1,2,3,4-tétrahydro-2,2-diméthylquinoléine-6-yl)propionique et ses sels acceptables en pharmacie.

4. Acide 2-(8-chloro-1,2-dihydro-1,2,2-triméthylquinoléine-6-yl)propionique et ses sels acceptables en pharmacie.

5. Acide 2-(8-fluoro-1,2-dihydro-2,2-diméthylquinoléine-6-yl)propionique et ses sels acceptables en pharmacie.

6. Acide 2-(8-fluoro-1,2,3,4-tétrahydro-2,2-diméthylquinoléine-6-yl)propionique et ses sels acceptables en pharmacie.

7. Acide 2-(8-fluoro-1,2-dihydro-1,2,2-triméthylquinoléine-6-yl)propionique et ses sels acceptables en pharmacie.

8. Acide 2-(1-allyl-1,2-dihydro-2,2-diméthylquinoléine-6-yl)propionique et ses sels acceptables en pharmacie.

9. Acide 2-(1,2-dihydro-1,2,2-triméthylquinoléine-6-yl)propionique et ses sels acceptables en pharmacie.

10. Un composé comme revendiqué dans l'une quelconque des revendications 1 à 9 pour l'emploi dans le traitement d'une maladie ou d'un état nécessitant un agent anti-inflammatoire antipyrétique ou analgésique.

11. Un composition comprenant un support et un composé comme revendiqué dans l'une quelconque des revendications 1 à 11.

**Revendications pour l'Etat contractant: AT**

1. Un procédé pour la préparation de composés d'acide quinolylacétique de formule générale (I)

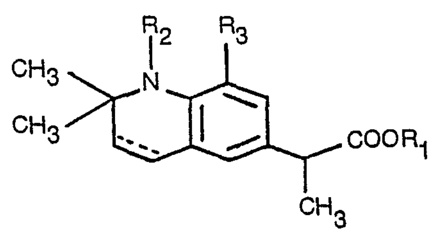

(I)

(dans laquelle le pointillé indique une double liaison éventuelle entre les positions 3 et 4; $R_1$ est un atome d'hydrogène ou un groupe alkyle $C_{1-4}$; $R_2$ est un atome d'hydrogène ou, lorsque le pointillé représente une double liaison, un groupe méthyle ou allyle; et $R_3$ est un atome d'halogène ou, lorsque le pointillé représente une double liaison, un atome d'hydrogène; sous réserve que lorsque le pointillé représente une double liaison, au moins un de $R_2$ et $R_3$ est autre qu'un atome d'hydrogène), et leurs sels acceptables en pharmacologie, que comprend

(a) la réaction d'un composé de formule

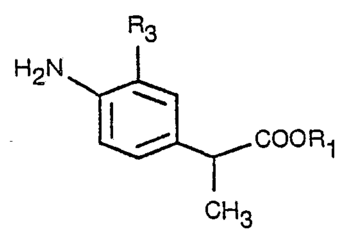

(II)

(dans laquelle $R_1$ et $R_3$ sont comme défini ci-dessus) avec un composé de formule

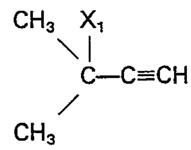

(III)

(dans laquelle $X_1$ représente un atome d'halogène) pour former un composé de formule

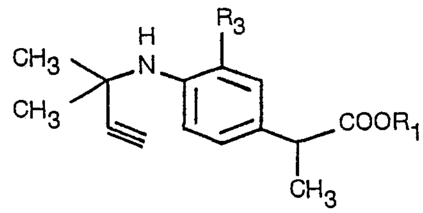

(IV)

(dans laquelle $R_1$ et $R_3$ sont comme défini ci-dessus) et la réalisation d'une cyclisation pour former un composé de formule générale (I);

(b) l'hydrogénation d'un composé de formule générale

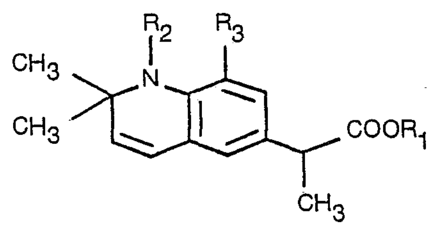

(I–2)

(dans laquelle $R_1$, $R_2$ et $R_3$ sont comme défini ci-dessus);

(c) la réaction d'un composé de formule

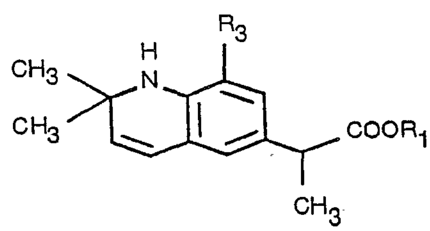

(I–4)

(dans laquelle $R_1$ et $R_3$ sont comme défini ci-dessus) avec un composé de formule

$$R_2^*—X_2 \quad (V)$$

(dans laquelle $R_2^*$ est comme défini ci-dessus pour $R_2$, si ce n'est qu'il n'est pas un atome d'hydrogène, et $X_2$ est un atome d'halogène, un groupe hydroxy ou un groupe ester actif); et

(d) la réduction d'un composé de formule

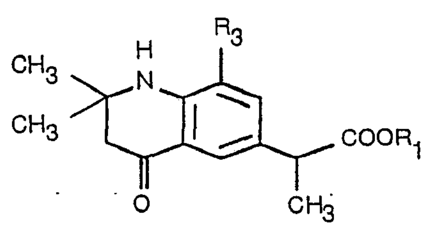

(I–8)

(dans laquelle $R_1$ et $R_3$ sont comme défini ci-dessus).

2. Un procédé selon la revendication 1 pour la préparation de l'acide 2-(8-chloro-1,2-dihydro-2,2-diméthylquinoléine-6-yl)propionique et de ses sels acceptables en pharmacie.

3. Un procédé selon la revendication 1 pour la préparation de l'acide 2-(8-chloro-1,2,3,4-tétrahydro-2,2-diméthylquinoléine-6-yl)propionique et de ses sels acceptables en pharmacie.

4. Un procédé selon la revendication 1 pour la préparation de l'acide 2-(8-chloro-1,2-dihydro-1,2,2-triméthylquinoléine-6-yl)propionique et de ses sels acceptables en pharmacie.

5. Un procédé selon la revendication 1 pour la préparation de l'acide 2-(8-fluoro-1,2-dihydro-2,2-diméthylquinoléine-6-yl)propionique et de ses sels acceptables en pharmacie.

6. Un procédé selon la revendication 1 pour la préparation de l'acide 2-(8-fluoro-1,2,3,4-tétrahydro-2,2-diméthylquinoléine-6-yl)propionique et de ses sels acceptables en pharmacie.

7. Un procédé selon la revendication 1 pour la préparation de l'acide 2-(8-fluoro-1,2-dihydro-1,2,2-triméthylquinoléine-6-yl)propionique et de ses sels acceptables en pharmacie.

8. Un procédé selon la revendication 1 pour la préparation de l'acide 2-(1-allyl-1,2-dihydro-2,2-diméthylquinoléine-6-yl)propionique et de ses sels acceptables en pharmacie.

9. Un procédé selon la revendication 1 pour la préparation de l'acide 2-(1,2-dihydro-1,2,2-triméthylquinoléine-6-yl)propionique et de ses sels acceptables en pharmacie.

10. Un procédé pour la préparation d'une composition pharmaceutique qui comprend le mélange d'un composé de formule (I) comme défini dans la revendication 1 ou d'un de ses sels acceptables en pharmacie avec un support ou excipient pharmaceutique.

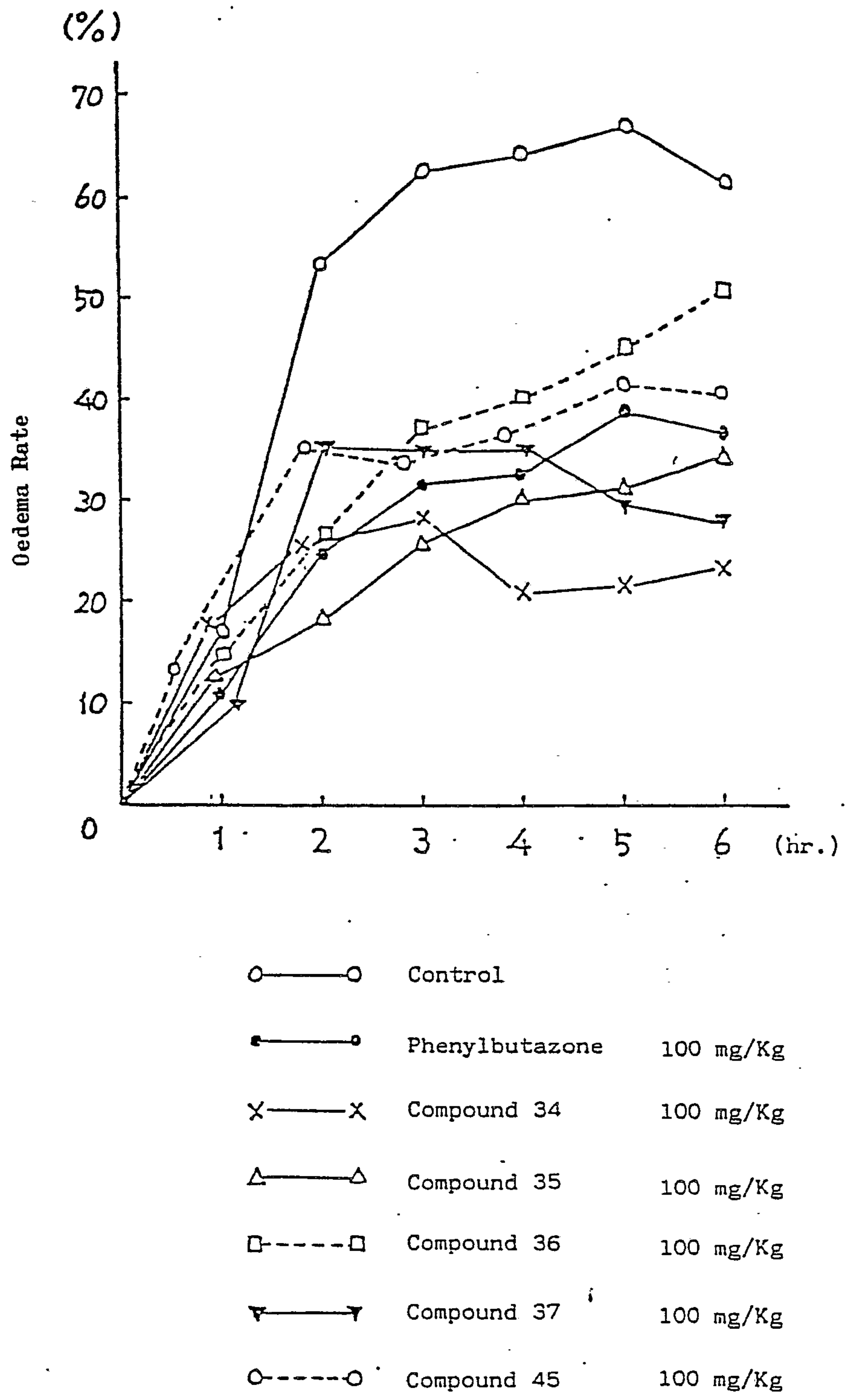
(%)
70
60
50
40
30
20
10
0
Oedema Rate
1
2
3
4
5
6
(hr.)
Control
Phenylbutazone 100 mg/Kg
Compound 34 100 mg/Kg
Compound 35 100 mg/Kg
Compound 36 100 mg/Kg
Compound 37 100 mg/Kg
Compound 45 100 mg/Kg